# EUROPEAN PATENT APPLICATION

(11) **EP 1 120 651 A1**
(43) Date of publication of application: **01.08.2001**
(21) Application number: 00949955.9
(22) Date of filing: 02.08.2000
(51) Int. Cl.: G01N 33/574

(54) **METHOD AND REAGENT FOR ASSAYING ARTHRITIS-ASSOCIATED MELANOTRANSFERRIN**

(30) Priority: 05.08.1999 JP 22256899
(71) Applicant: ORIENTAL YEAST CO., LTD., Tokyo 174-8505 (JP)
(72) Inventor: KATO, Yukio, Hiroshima-shi, Hiroshima 732-0062 (JP); MATSUKAWA, Hirokazu, Itabashi-ku, Tokyo 174-8505 (JP); YOSHIWARA, Yutaka, Itabashi-ku, Tokyo 174-8505 (JP); OKA, Osamu, Itabashi-ku, Tokyo 174-8505 (JP); FUJITA, Tuyosi, Itabashi-ku, Tokyo 174-8505 (JP)
(74) Representative: HOFFMANN - EITLE
(86) International application number: JP0005171
(87) International publication number: WO0111368

(57) **Abstract**

The present invention aims to provide a method for assaying arthritis-related melanotransferrin as well as a reagent and a kit for use in said method. The method of the present invention comprises;
i) measuring the melanotransferrin level in a biological sample; and
ii) indicating arthritis if the melanotransferrin level in the biological sample is significantly higher than in a control non-arthritis biological sample.

## Description

### TECHNICAL FIELD

The present invention relates to methods and reagents for assaying arthritis-related melanotransferrin.

### BACKGROUND ART

### Arthritis

Inside joints, a joint cavity is formed between bones covered with cartilage and filled with synovial fluid. Synovial membrane is surrounded by a joint capsule, bones, muscles, tendons, ligaments or the like. Joints are liable to inflammation because they are always subjected to mechanical stimulation from motion. Inflammation in joints is generally called "arthritis" (Medical Dictionary, 18th ed., p. 385, 1998, Nanzando).

### i) Osteoarthritis

Among various types of arthritis, "osteoarthritis (OA, osteoarthrosis)" also called arthrosis deformans is a disease characterized by both chronic degenerative changes and proliferative changes in joints leading to morphologic changes of the joints. It is largely classified as primary and secondary OA. The former mostly occurs after middle age, 80% thereof among women. It is caused by senescence combined with physical stress. The latter is found even in juveniles and sequently occurs after known causes such as joint trauma, malformation, diseases, dysbolism, etc. Pathologically, articular cartilage is gradually worn or lost whereby bone is exposed. At the same time, cartilage auxetic growth and spur formation occur with vascular proliferation. Other pathologies include synovial membrane growth, joint capsule thickening or atrophy, appearance of loose bodies, etc. Symptoms include joint stiffening followed by pain upon movement, limitation of range of movement and joint swelling. Sometimes, pain or a creaking sound occurs when movement is started. Radiography shows spur formation, joint gap narrowing or disappearance, subchondral bone induration, sacculation, etc. Treatment is provided by conservative therapies such as physiotherapy, kinesitherapy, orthotic therapy or medical therapy. Advanced legions with serious dysfunction are surgically treated (Medical Dictionary, 18th ed., p. 1926, Nanzando, supra).

### ii) Rheumatoid arthritis

Rheumatoid arthritis (RA), also called chronic rheumatoid arthritis, is a disease characterized by chronic arthritis of unknown etiology. The disease occurs more frequently in women with a female to male ratio of 1:4 and begins to occur most commonly among people in between their 30s and 50s. It is presumed to occur in from about 0.3% to 0.5% of the overall population in Japan. Although the cause remains unknown, multifactorial hereditary predisposition, especially in relation to HLA-D4 and virus infection, is noted. The disease is typically a symmetric polyarthritis and always affects synovial joints. The earliest change is only inflammatory swelling of the synovial membrane, but as the disease advances, cartilage and bone are destroyed and joints are deformed or dislocated or lose mobility due to osseous ankylosis. Especially, fingers and toes are liable to deformation characterized by ulnar displacement at metacarpophalangeal joints, swan neck deformity or button hole deformity in fingers, and hallux valgus or the like in toes. The feeling of malfunction or stiffness in joints when waking up in the morning is a symptom called "morning stiffness", which is important for diagnosis and follow-up. Extra-articular manifestations include vasculitis, pericarditis, subcutaneous nodules, lung fibrosis, etc., among which the type accompanied by vasculitis may show a poor prognosis resembling polyarteritis nodosa to induce malignant rheumatoid arthritis.

Clinical tests detect rheumatoid factors as high as 70%-80% together with increased erythrocyte sedimentation, positive CRP, mild anemia, increased platelets, high serum complement titer, etc. Findings in synovial tissues include non-specific chronic synovitis.

Treatment is given by combining internal therapy using anti-inflammatory agents and remission introducers with orthopedic therapy, rehabilitation, etc. (Medical Dictionary, 18th ed., p. 2019, Nanzando, supra).

### iii) Traumatic joint injury

Among various types of arthritis, joint injury caused by extraneous mechanical or physical stimulation is called "traumatic joint injury (ACL injury)". More specifically, this is a condition in which joints are mechanically or physically broken and cartilage tissues are also destroyed by a traffic accident or the like, with the result that constitutive protein in cartilage tissues transiently infiltrates into synovial fluid.

### Diagnosis of arthritis

Conventionally, arthritis has been primarily diagnosed by radiographically detecting injury and wear at joints. However, destruction and injury in cartilage tissues of joints can be radiographically detected only at substantially advanced or aggravated stages. This is disadvantageous for early diagnosis/treatment of arthritis.

For diagnosis of rheumatoid arthritis, the criteria recommended by the American Rheumatism Association are widely used. Specifically, patients are diagnosed as suffering from rheumatoid arthritis if their symptoms fall under four or more of seven criteria including morning stiffness, symmetrical polyarthritis, subcutaneous nodules, radiological findings in hand joints, etc. as described above. The diagnosis based on these criteria may not always be sufficient from the viewpoint of convenience and early diagnosis of arthritis.

Several extracellular matrix proteins such as laminin or osteonectin have been found as proteins present in cartilage tissues in joints. Laminin is a glycoprotein constituting a main component of basal membrane and has a role of adhering epithelial cells to connective tissues and promoting elongation of neurites. Osteonectin is an acidic phosphoglycoprotein occupying about 1/4 of non-collagenic proteins of bone. It was discovered from organic matrix of bone, and later shown to be widely present in soft tissues other than bone tissues (Dictionary of Biochemistry, 3rd ed., p.1464 and p. 251, October, 1998). Makamura et al. (ARTHRITIS & RHEUMATISM Vol. 39, No. 4, 1996, pp. 539-551) suggests that osteonectin levels in human synovial fluid are related to rheumatoid arthritis. However, it also describes that osteonectin shows high levels in rheumatoid arthritis but is unsuitable for the diagnosis of osteoarthritis. Further, the relation with other types of arthritis has been unknown.

Thus, no marker indicative of a wide range of arthritis has existed and also it had been impossible to discriminate among osteoarthritis, rheumatoid arthritis and traumatic joint injury prior to the present invention.

### Melanotransferrin

"Melanotransferrin" (hereinafter referred to as "MTF") is a protein identified as tumor-associated antigen "p97" from human melanoma cells (Brown et al., 1980, J. Biol. Chem. 255, pp. 4980-4983; Dipplod et al., 1980, Proc, Natl. Acad. Sci., USA, 77, pp. 6114-6118; Woodburg et al., 1980, Proc. Natl. Acad. Sci., USA, 77, pp. 2183-2187). MTF antigen has been widely investigated for the expression in normal and tumor tissues and known to occur in most human melanoma cells (Brown et al., 1981, J. Immonuol. 127, pp. 539-546; Brown et al., 1981, Proc. Natl. Acad. Sci., USA, 78, pp. 539-543; Garrigues et al., 1982, Int. J. Cancer, 29, pp. 511-515). Therefore, it is used as a target for diagnostic imaging of tumor in human clinical experiences (Larson et al., 1983, J. Clin. Invest., 72, pp. 2101-2114).

MTF was reported to also occur in hepatic cells (Sciot et al., Liver, Vol. 9 (1989), pp. 110-119), fetal intestinal epithelial cells (Danielsen et al.. J. Cell Biol., Vol. 131 (1995), pp. 939-950) and brain (Rothenberger et al., Brain Research 712 (1996) pp. 117-121).

Recently, MTF was suggested to be responsible for iron intake into brain cells and found to be present in patients with Alzheimer's disease at levels several times higher than in normal individuals. Based on these results, diagnostic agents for Alzheimer's disease via MTF assay are under development (Nikkei Biotech, p. 11, March, 29, 1999).

Kawamoto et al. (Eur. J. Biochem., 1998, Sep. 15, 256 (3), pp. 503-509) reported the expression of rabbit MTF protein on the surfaces of rabbit cartilage cells. However, neither the expression of MTF protein on human cartilage cells nor the presence of MTF protein in synovial fluid has been disclosed. The presence of MTF in joint-associated tissues or fluids has not been reported either. Further. the relation between arthritis and MTF was unknown prior to the present invention.

### SUMMARY OF THE INVENTION

An object of the present invention is to provide an assay method for arthritis-related melanotransferrin. The assay method of the present invention comprises;
i) measuring the melanotransferrin level in a biological sample; and
ii) indicating arthritis if the melanotransferrin level in the biological sample is significantly higher than in a control non-arthritis biological sample.

In an embodiment, the method of the present invention comprises indicating arthritis if the melanotransferrin level in synovial fluid is about 2 ng/ml or more or if the melanotransferrin level in serum is about 5 ng/ml or more.

In an embodiment, the method of the present invention comprises indicating any one of rheumatoid arthritis, osteoarthritis (OA) and traumatic joint injury if the melanotransferrin level in synovial fluid is about 2 ng/ml or more, indicating rheumatoid arthritis or traumatic joint injury if it is about 8 ng/ml or more, and indicating rheumatoid arthritis if it is about 25 ng/ml or more.

In an embodiment, the method of the present invention comprises measuring the melanotransferrin level in a biological sample by a sandwich immunoassay using a melanotransferrin-specific antibody.

Another object of the present invention is to provide a reagent for use in said assay method for melanotransferrin comprising a melanotransferrin-specific antibody.

Another object of the present invention is to provide a kit for assaying melanotransferrin comprising said reagent in a suitable container.

### BRIEF DESCRIPTION OF DRAWINGS

FIG. 1 shows a calibration curve of a standard melanotransferrin solution used in a sandwich enzyme immunoassay of the present invention.

FIG. 2 shows MTF levels in synovial fluid from patients with various types of arthritis measured by a sandwich enzyme immunoassay of the present invention.

FIG. 3 shows the correlation between MTF levels in synovial fluid and MTF levels in serum found by a sandwich enzyme immunoassay of the present invention.

### DETAILED DESCRIPTION OF THE INVENTION

The present inventors conducted intensive studies to solve the above-described problems. As a result, they have successively found that MTF levels are related to arthritis thereby completing the present invention. Specifically, the present invention first revealed that MTF can be an indicator of a wide range of arthritis and further even discriminate among osteoarthritis, rheumatoid arthritis and traumatic joint injury.

Specifically, the present invention relates to an assay method for arthritis-related MTF comprising indicating arthritis if the MTF level in a biological sample is significantly higher than in a control non-arthritis biological sample.

### MTF

As used herein, "MTF" refers to a tumor-associated antigen first identified in human melanoma cells, as described above. It is a monomer of cell surface sialoglycoprotein having an apparent molecular weight (MW) of about 97,000 Da measured by sodium dodecyl sulfate-polyacrylamide gel electrophoresis (SDS-PAGE). MTF protein is one of proteins belonging to the transferrin family having an iron binding ability and considered to be a molecule responsible for endocytosis. More specifically, MTF protein shares homologous amino acid sequences with transferrin and binds iron similarly to transferrin (Brown et al., 1982, Nature, London, 296, pp. 171-173). Somatic hybrid analysis and in situ hybridization showed that the MTF gene is located on a chromosomal region between 3q21 and 3q29 similarly to the transferrin gene (Prowman et al., 1984, Proc. Natl. Acad. Sci., USA, 81, pp. 2752-2756).

Japanese Patent Public Disclosure Nos. 280390/96 and 135692/97 incorporated herein as references disclose the nucleotide sequence and putative amino acid sequence of human MTF. The amino acid sequence of human MTF (718 aa) is shown herein as SEQ ID NO: 1 together with the coding nucleotide sequence.

### Assay methods

In assay methods of the present invention, MTF in a biological sample can be measured by using any known techniques.

Preferably, MTF protein can be measured by immunoassays. Immunoassays involve detecting an antigen or antibody based on an antigen-antibody reaction in which an antibody specifically recognizes an antigen. In immunoassays, a wide variety of detection markers such as enzymes, radioactive tracers, chemiluminescent or fluorescent substances, metal atoms, sols, latexes and bacteriophages can be used.

Among immunoassays, enzyme immunoassays (EIA) using an enzyme are widely used as especially excellent assays in terms of economy and convenience. An outstanding review on enzyme immunoassays is found in Tijssen P, "Practice and theory of enzyme immunoassays" in Laboratory techniques in biochemistry and molecular biology, Elsevier Amsterdam New York, Oxford ISBN 0-7204-4200-1 (1990).

In the present invention, procedures for preparing and immunochemically assaying antigens and antibodies are not specifically limited but any known techniques can be used. The preparation can be performed by known procedures as described in "Textbook of Biochemical Experiments, Vol. 2., Methods in Immunology and Biochemistry", The Japanese Biochemical Society ed., for example. Embodiments of procedures for preparing and immunochemically assaying antigens and antibodies of the present invention are illustrated below.

### i) Preparation of antibodies

Polyclonal antibodies used in assay methods of the present invention can be routinely prepared.

First, an MTF immunogen is prepared. For example, membrane fragments are initially prepared from human cartilage cultured cells and then membrane-bound components are solubilized with phospholipase C specifically acting on GPI anchors. Then, the solubilized components are applied on an MTF-binding lectin-immobilized gel so that MTF is adsorbed to the lectin gel. Glycoprotein fragments adsorbed to the lectin gel are eluted by using pH changes, salinity changes or a specific sugar (for example, α-methylmannopyranoside when the immobilized lectin is concanavalin A). Then, an MTF antigen can be purified by ion exchange column chromatography, gel filtration chromatography or the like, if desired. Alternatively, an MTF antigen can be prepared by affinity chromatography using a known anti-MTF monoclonal antibody (for example, L235 (ATCC No. 8446-HB), etc.). Suitable immunogens may be derived from any sources including human and non-human mammals such as rabbit. Preferably, immunogens are derived from human.

Alternatively, the MTF gene can be cloned on the basis of the base sequences of the MTF gene disclosed in Japanese Patent Public Disclosure Nos. 280390/96 and 135692/97 or the like to express MTF protein using known genetic engineering techniques.

Thus obtained MTF immunogen is dissolved in a sodium phosphate buffer (hereinafter referred to as "PBS") and mixed with a solubilizer such as Freund's complete or incomplete adjuvant or alum, and then administered to an animal as an immunizing antigen. Suitable animals to be immunized include any species routinely used in the art such as mouse, rat, rabbit, goat, horse, etc.

Immunization can be performed by any of subcutaneous injection, intraperitoneal injection, intravenous injection, intracutaneous injection or intramuscular injection, preferably by subcutaneous injection or intraperitoneal injection. Immunization can be performed once or plural times at appropriate intervals, preferably at intervals of 1-5 weeks.

Blood is collected from the immunized animal and serum is separated to evaluate the presence of a polyclonal antibody reacting MTF.

Monoclonal antibodies of the present invention can also be prepared by known procedures.

For preparing a monoclonal antibody, at least the following steps are needed:
(a) preparation of MTF as an antigen for immunization.
(b) immunization of an animal,
(c) collection of blood, assay and preparation of antibody-producing cells,
(d) preparation of myeloma cells,
(e) cell fusion between the antibody-producing cells and the myeloma cells and selective culture of hybridomas,
(f) screening of hybridomas producing a target antibody and cell cloning.
(g) preparation of a monoclonal antibody by culturing the hybridomas or implanting the hybridomas into an animal,
(h) determination of the reactivity of the prepared monoclonal antibody.

Standard procedures for preparing hybridomas producing monoclonal antibodies are described in, for example, "Hybridoma Techniques", Cold Spring Harbor Laboratory, 1980; "Cytohistochemistry", Yamamoto et al., The Japan Society of Histochemistry and Cytochemistry ed., Gakusai Kikaku, 1986.

A procedure for preparing a monoclonal antibody against MTF of the present invention is explained below. However, as will be obvious to those skilled in the art, procedures of the present invention are not limited thereto.

Steps (a)-(b) can be performed essentially in the same manner as described for polyclonal antibodies.

At step (c), suitable antibody-producing cells are lymphocytes typically derived from spleen, thymus, lymph nodes, peripheral blood or a combination thereof, most typically spleen cells. Thus, an organ containing antibody-producing cells such as spleen is extracted from a mouse shown to produce antibodies after final immunization, whereby spleen cells are prepared.

At step (d), suitable myeloma cells include, for example, Balb/c mouse-derived myeloma cell lines such as P3/X63-Ag8 (X63) (Nature, 256, 495-497 (1975)), P3/X63-Ag8.U1 (P3U1) (Current Topics. in Microbiology and Immunology, 81, 1-7 (1987)), P3/NSI-1-Ag 4-1 (NS-1) (Eur. J. Immunol., 6, 511-519 (1976)), Sp2/0-Ag14 (Sp2/0) (Nature, 276, 269-270 (1978)), FO (J. Immuno. Meth., 35, 1-21 (1980)), MPC-11, X63.653, S194 or rat-derived myeloma cell lines such as 210.RCY3.Ag1.2.3. (Y3) (Nature, 277, 131-133, (1979)).

Said myeloma cells are successively cultured in Dulbecco's modified Eagle's medium (DMEM) or Iscove's modified Dulbecco's medium (IMDM) containing fetal calf serum to ensure about 1 x 10⁶ or more cells on the day of fusion.

Cell fusion at step (e) can be performed according to known methods as described by Milstein et al. (Methods in Enzymology, 73, 3 (1981)), for example. Methods using polyethylene glycol (PEG) are currently most common. PEG methods are described in, for example, "Cytohistochemistry", Yamamoto et al. supra. As an alternative fusion technique, electric treatment (electric fusion) may also be used (Okouchi et al., Experimental Medicine, 5, 1315-19, 1987). Other methods may also be appropriately used. The ratio of cells used may also be similar to those used in known techniques, such as 3-10:1 of spleen cells to myeloma cells.

Selection of hybridomas that acquired antibody-secreting ability and proliferation ability as a result of fusion of spleen cells and myeloma cells can be performed using HAT medium prepared by adding hypoxanthine aminopterin thymidine to said DMEM or IMDM when a hypoxanthine-guanine phosphoribosyltransferase-deficient strain is used as a myeloma cell strain, for example.

At step (f), the culture supernatant containing selected hybridomas is partially removed and measured for the antibody activity against MTF by an ELISA method described later, for example.

Subsequently, hybridomas shown to produce antibodies reacting to MTF are cloned. Suitable methods for this cell cloning include "limiting dilution analysis" involving diluting a mixture of hybridoma cells until one hybridoma cell is contained in each well; soft agar cloning; isolating a single cell with a mioromanipulator; "sorter cloning" involving separating a single cell with a cell sorter; etc. Limiting dilution is simple and common.

Wells showing antibody titer are subjected to 1-4 runs of cloning by limiting dilution, for example, to select those stably showing antibody titer as anti-MTF monoclonal antibody-producing hybridoma strains. Suitable media for culturing hybridomas include, for example, DMEM or IMDM containing fetal calf serum (FCS). Hybridomas are preferably cultured at a carbon dioxide concentration of about 5-7% and 37°C (in an incubator at 100% humidity), for example.

Mass culture for preparing antibodies at step (g) is performed in a hollow fiber or similar culture apparatus. Alternatively, hybridomas can be grown in the abdominal cavity of inbred mouse (for example, Balb/c supra) or Nu/Nu mouse to prepare antibodies from ascites.

Thus obtained culture supernatants or ascites can be used as anti-MTF monoclonal antibodies, or further subjected to salting out with ammonium sulfate, gel filtration, lyophillization or the like and antibody fragments may be collected and purified to give anti-MTF monoclonal antibodies. When purification is needed, conventional techniques such as ion exchange column chromatography, affinity chromatography, high-speed liquid chromatography (HPLC) can be used in combination.

Thus obtained anti-MTF monoclonal antibodies can be determined for their subclass, antibody titer or the like using known methods such as ELISA methods described later.

In the present invention, novel anti-MTF monoclonal antibodies 47-4E and 8-9C were obtained as described later in the examples. As shown in Table 1 of Example 1, 47-4E and 8-9C are monoclonal antibodies highly specific for MTF, which have no cross-reactivity to transferrin, which resembles MTF, Anti-MTF monoclonal antibodies known prior to the present invention and deposited with American Type Culture Collection such as L235 (ATCC No. 8446-HB) can also be used.

The aforementioned paper of Kawamoto et al. (Eur. J. Biochem., 1998) discloses an antiserum to rabbit MTF. Such an antiserum can also be used in assay methods of the present invention directed to rabbit, for example.

### Antibody-linked assays of MTF in biological samples

Antibody-linked assays of MTF used herein include various techniques commonly used for the detection of antigens such as radioimmunoassays (RIA), ELISA methods (Engvall, E., Methods in Enzymol., 70, 419-439 (1980)), fluorescent antibody techniques, plaque techniques, spot techniques, agglutination, Ouchterlony methods, etc. ("Hybridoma Techniques and Monoclonal Antibodies", R&D Planning, pp. 30-53, March 5, 1982).

For example, MTF assays can be performed according to the following protocol by an indirect competitive ELISA method among various ELISA methods:
(a) First, immobilizing an MTF antigen on a support.
(b) Blocking the solid-phase surface on which the antigen has not been adsorbed with an antigen-unrelated substance, such as a protein.
(c) Adding biological samples containing MTF at various levels and antibodies, and competitively reacting anti-MTF antibodies with said immobilized antigen and MTF to produce immobilized antigen-antibody complexes and MTF-antibody complexes.
(d) Measuring the amounts of the immobilized antigen-antibody complexes to determine the amounts of MTF in the biological samples from a preliminarily prepared calibration curve.

At step (a), the support on which an antigen is to be immobilized is not specifically limited, but may be any of those normally used in ELISA methods such as a 96-well polystyrene microtiter plate.

For immobilizing the antigen on a support, a buffer containing the antigen can be incubated on the support, for example. Known buffers such as phosphate buffers are suitable. The concentration of the antigen in the buffer can be selected in a wide range, but suitably ranges from normally 0.01 µg/ml to 100 µg/ml, preferably 0.05 µg/ml to 10 µg/ml. When a 96-well microtiter plate is used as a support, the density is 300 µl/well or less, desirably from 20 µl/well to 150 µl/well. Incubation conditions are not specifically limited, but suitably incubation is normally performed at 4°C overnight.

At step (b), the only purpose of blocking is to prevent any post-added antibodies from being adsorbed in addition to MTF to the support on which the antigen has been immobilized. Suitable blocking agents include, for example, BSA or skimmed milk solution. Alternatively, commercially available blocking agents such as "Block Ace" from Dainippon Pharmaceutical. Code No. UK-25B, are also suitable. As a specific but non-limitative example, an appropriate amount of a buffer (for example, a 85 mM borate buffer (pH 8.0) supplemented with 1% BSA and 60 mM NaCl) containing a blocking agent can be added to the antigen-immobilized support, and incubated at about 4°C for 1 hour to 5 hours, and then washed with a washing solution. Suitable washing solutions include, but are not specifically limited to, PBS, for example.

At step (c), biological samples containing MTF and antibodies are then brought into contact with the immobilized antigen whereby the antibodies are reacted with the immobilized antigen and MTF to produce immobilized antigen-antibody complexes and MTF-antibody complexes.

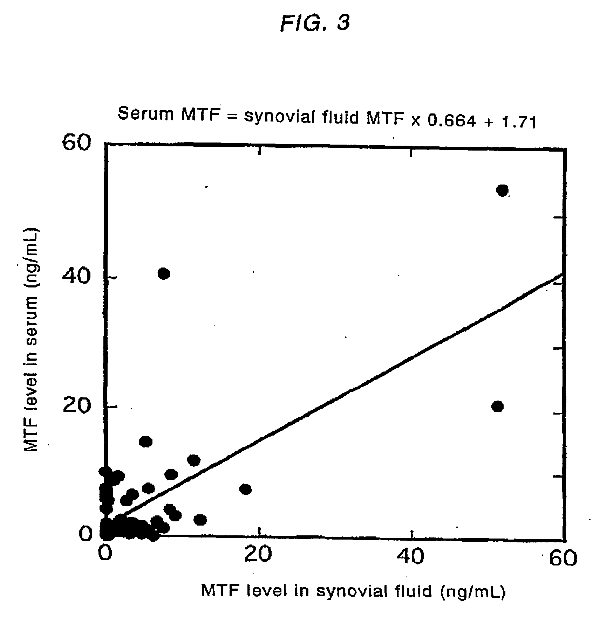

Suitable biological samples containing MTF include, but are not specifically limited to, synovial fluid and serum or the like. As shown in Example 4 (Fig. 3) below, MTF levels in synovial fluid have a correlation with MTF levels in serum. Therefore, synovial fluid is preferably used in assay methods of the present invention, but other biological samples such as serum, blood, lymphocytes may also be used. Suitable biological samples may be derived from any sources including human and non-human mammals such as rabbit. Preferably, biological samples are derived from human.

As for antibodies, a first antibody against MTF of the present invention and then a marker enzyme-coupled second antibody against the first antibody are successively added to react them.

The first antibody is added as a solution in a buffer. As a non-limitative example, the reaction may be performed at 10°C - 40°C, preferably about 25°C for about 1 hour. After completion of the reaction, the support is washed with a buffer to remove the first antibody unbound to the immobilized antigen. Suitable washing solutions include, for example, PBS.

Then, the second antibody is added. For example, when the first antibody is a mouse monoclonal antibody, an antimouse-goat antibody to which a marker such as an enzyme has been coupled is suitably used. Suitable markers include enzymes such as horseradish peroxidase (hereinafter referred to as "HRP") or alkaline phosphatases. Alternatively, fluorescent substances such as fluorescein isocyanate or rhodamine, radioactive substances such as ³²P or ¹²⁵I or chemiluminescent substances can also be used. Desirably, a second antibody preferably diluted with a buffer to a final absorbency of 4 or less, more preferably 0.5-3.0 is reacted with a first antibody coupled to a support. As a non-limitative example, the reaction is performed at room temperature for about 1 hour followed by washing with a buffer. As a result of the reaction, the second antibody is bound to the first antibody. Alternatively, a labeled first antibody may be used, in which case a second antibody is unnecessary. Alternatively, a biotinylated first antibody may be combined with labeled streptavidin in place of a second antibody.

At step (d). a chromogenic substrate solution which can react with the marker of the second antibody coupled to the support, is then added and the absorbency is measured to calculate the amounts of MTF from a calibration curve.

When peroxidase is used as an enzyme to be coupled to the second antibody, a chromogenic substrate solution containing hydrogen peroxide and 3,3',5,5'-tetramethylbenzidine or o-phenylenediamine (hereinafter referred to as "OPD") can be used, for example. As a non-limitative example, the chromogenic substrate solution is added to perform a reaction at room temperature for about 10 minutes, and then the enzymatic reaction is stopped by adding 1N sulfuric acid. When 3,3'.5,5'-tetramethylbenzidine is used, the absorbency at 450 nm is measured. When OPD is used, the absorbency at 492 nm is measured. When an alkaline phosphatase is used as an enzyme to be coupled to the second antibody, however, a suitable procedure involves using p-nitrophenylphosphate as a chromogenic substrate, stopping the reaction with a 1 N NaOH solution and measuring the absorbency at 415 nm.

The decrease in the absorbency of a solution containing MTF reacted with antibodies as compared with a reaction solution without MTF is calculated as an inhibition rate. The MTF level in a sample can be calculated using a calibration curve preliminarily prepared from the inhibition rate of a reaction solution containing a known level of MTF.

Alternatively, MTF can also be assayed by a direct competitive ELISA method as below.
(a) First, immobilizing an anti-MTF monoclonal antibody on a support.
(b) Blocking the surface of the support on which the antibody has not been immobilized with an antigen-unrelated substance, such as a protein.
(c) Separately from said steps, preparing mixtures from biological samples containing MTF at various levels added with an enzyme-coupled MTF immunogen.
(d) Reacting said mixtures with said antibody-immobilized support.
(e) Measuring the amounts of immobilized antibody-enzyme-coupled MTF complexes to determine the amounts of MTF in the biological samples from a preliminarily prepared calibration curve.

At step (a), the support on which a monoclonal antibody is to be immobilized is not specifically limited but may be any of those normally used in ELISA methods such as a 96-well polystyrene microtiter plate. For immobilizing a monoclonal antibody, a buffer containing the monoclonal antibody can be incubated on the support, for example. The composition and the concentration of the buffer may be similar to those described above for the indirect competitive ELISA method.

At step (b), the purpose of blocking is to prevent any MTF in post-added samples and enzyme-coupled MTF from being adsorbed irrespective of antigen-antibody reaction to the support on which the antibody has been immobilized. Blocking agents and techniques may be similar to those described above for the indirect competitive ELISA method.

The enzyme-coupled MTF used at step (c) can be prepared by any method for coupling MTF to an enzyme. For example, the activated ester method described above can be used. The thus prepared enzyme-coupled MTF is mixed with samples containing MTF.

At step (d), the samples containing MTF and enzyme-coupled MTF are brought into contact with the antibody-immobilized support so that MTF and enzyme-bound MTF undergo a competitive inhibition reaction to produce complexes with the immobilized antibody. The samples containing MTF are used as dilutions in an appropriate buffer. As a non-limitative example, the reaction is performed at room temperature for about 1 hour. After completion of the reaction, the support is washed with a buffer to remove enzyme-coupled MTF unbound to the immobilized antibody. Suitable washing solutions include, for example, PBS.

At step (e), a chromogenic substrate solution reacting with the enzyme of enzyme-coupled MTF is then added similarly to the indirect competitive ELISA method described above and the absorbency is measured to calculate the amounts of MTF from a calibration curve.

Assay methods of the present invention can also be preferably performed by using, but not limited to, sandwich methods. Sandwich methods are non-competitive immunoassays also called "double antibody methods". Sandwich methods are characterized by assaying an antigen with two distinct antibodies against non-overlapping epitopes on the antigen (Dictionary of Biochemistry, 3rd ed., p. 599, October, 1998).
(a) First, immobilizing a first anti-MTF monoclonal antibody on a support.
(b) Blocking the surface of the support on which the antibody has not been immobilized with an antigen-unrelated substance, such as a protein.
(c) Reacting biological samples containing MTF with said antibody-immobilized support.
(d) Adding and reacting a labeled second anti-MTF monoclonal antibody.
(e) Measuring the amounts of immobilized first anti-MTF monoclonal antibody-MTF-labeled second monoclonal antibody complexes to determine the amounts of MTF in the samples from a preliminarily prepared calibration curve.

At step (e), a chromogenic substrate solution reacting with the enzyme of the labeled second monoclonal antibody is added similarly to the ELISA methods described above and the absorbency is measured to calculate the amounts of MTF from a calibration curve. Alternatively, a biotinylated second antibody may be combined with labeled streptavidin to produce a biotin-avidin conjugate, thus allowing the marker enzyme coupled to avidin to develop color.

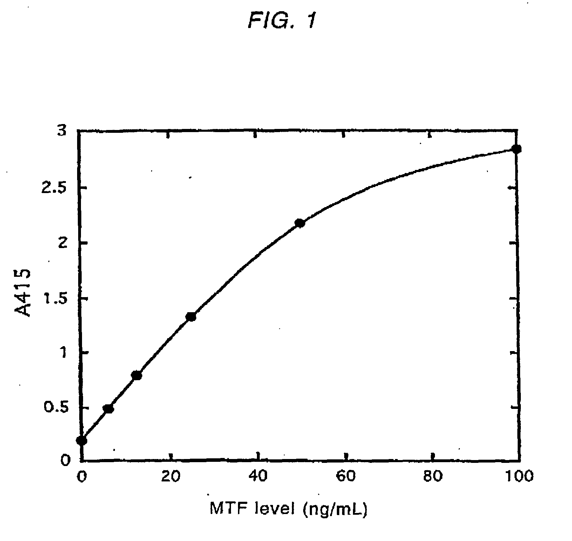

Sandwich methods are characterized in that the first monoclonal antibody immobilized on a support used at step (a) and the second monoclonal antibody added at step (e) recognize epitopes which are distinct from each other. Thus, the specificity and sensitivity for antigen detection can be increased. As a non-limitative example, anti-MTF monoclonal antibodies 47-4E and 8-9C obtained herein can preferably be used. In a sandwich method using anti-MTF monoclonal antibodies 47-4E and 8-9C, MTF can be measured at a level of about 1 ng/ml to about 80 ng/ml, preferably about 3 ng/ml to 60 ng/ml (Example 2, Fig. 1).

MTF is reported to be localized as a cell membrane protein and to have iron-binding ability and lectin-binding ability or the like (Rothenberger et al., Brain Research 712 (1996), pp. 117-121, etc.). Therefore, not only sandwich immunoassays using two antibodies, but also lectin-antibody sandwich assays can be used. Moreover, sandwich assays using antibody-iron labeled with a radioisotope such as ⁵⁷Fe, ⁵⁰Fe can also be used. In addition, the present invention is not limited to immunoassays, and any methods capable of measuring MTF levels in biological samples can be applied. Arthritis

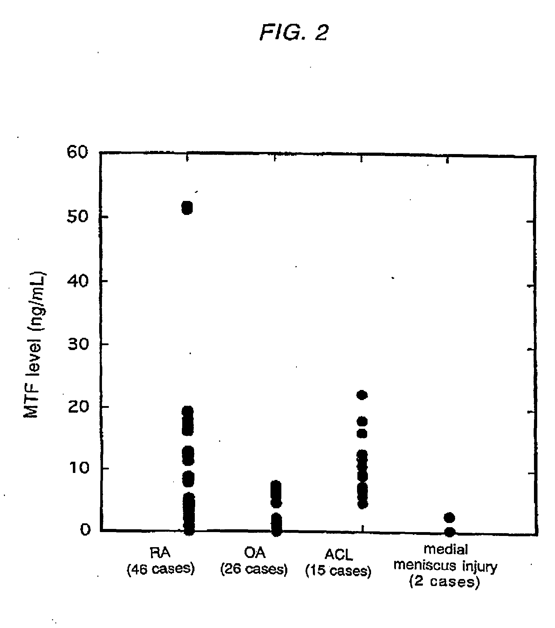

Assay methods of the present invention indicate arthritis if the MTF level in a biological sample is significantly higher than in a control non-arthritis biological sample. When MTF levels in a plurality of biological samples with various types of arthritis are measured by assay methods of the present invention, the MTF levels are always significantly higher than in a control non-arthritis biological sample. In Example 3 (Fig. 2) below, for example, MTF levels in synovial fluid were elevated by about 5-fold in osteoarthritis, about 10-fold in rheumatoid arthritis and about 7-fold in traumatic joint injury, compared with a negative control group (medial meniscus injury).

As a non-limitative example, assay methods of the present invention indicate arthritis if MTF level in synovial fluid is about 2 ng/ml or more. As shown in Example 4 (Fig. 3), there is a correlation between MTF levels in serum and MTF levels in synovial fluid with a correlation coefficient of 0.694 and the relational expression: serum MTF = synovial fluid MTF x 0.664 + 1.71. Thus, arthritis is indicated if MTF is about 5 ng/ml or more when the sample is serum.

The types of arthritis that can be indicated by assay methods of the present invention are not specifically limited, but include any known types of arthritis such as rheumatoid arthritis, osteoarthritis, traumatic joint injury, infective arthritis, connective tissue disease-associated arthritis, etc. Preferably, rheumatoid arthritis, osteoarthritis and traumatic joint injury can be targeted.

Assay methods of the present invention permit discrimination among the three types of arthritis, i.e. rheumatoid arthritis, osteoarthritis and traumatic Joint injury. Specifically, the methods indicate any one of rheumatoid arthritis, osteoarthritis and traumatic joint injury if MTF level in synovial fluid is about 2 ng/ml or more, indicating rheumatoid arthritis or traumatic joint injury if it is about 8 ng/ml or more, and indicating rheumatoid arthritis if it is about 25 ng/ml or more. If it is about 2 ng/ml or less, non-arthritis is indicated.

The indication of arthritis and discrimination among the three types of arthritis, i.e. rheumatoid arthritis, osteoarthritis and traumatic joint injury can be easier and more reliable by combining assay methods of the present invention with other data on arthritis such as radiographic data, criteria for the diagnosis of rheumatoid arthritis, osteonectin distribution data or the like as described above.

The present invention first revealed the relation between MTF and arthritis to permit early diagnosis and finding of arthritis. Therefore, it is thought that not only MTF protein levels in biological samples but also gene expression may be related to arthritis on the basis of the present invention. That is, the mRNA of MTF in biological samples can be amplified by using nucleic acid amplification techniques such as PCR and compared with a control sample from non-arthritis.

### Reagents and kits for assaying MTF

The present invention also provides a reagent for use in said MTF assay methods. The reagent of the present invention is used to measure MTF to diagnose arthritis. Moreover, the reagent of the present invention permits discrimination among the three types of arthritis, i.e. rheumatoid arthritis, osteoarthritis and traumatic joint injury.

The reagent of the present invention is characterized by comprising an MTF-specific antibody for measuring MTF levels. The MTF-specific antibody is preferably a monoclonal antibody, more preferably anti-MTF monoclonal antibodies 47-4E and 8-9C obtained by Example 1 of the present invention.

The present invention also provides a kit comprising said reagent in a suitable container. The MTF-specific antibody is preferably a monoclonal antibody. As a non-limitative example, the kit of the present invention comprises a unit of 10 µl to 10 ml of a solution containing an anti-MTF monoclonal antibody at a concentration of 10 µg/ml to 1,000 µg/ml in a container.

The kit of the present invention may further comprise a plate for immobilizing an antibody, a chromogenic substrate solution, a standard labeled antibody or a combination of a biotin-labeled antibody and a streptavidin- or avidin-labeled enzyme, and a reagent necessary for immunoassay such as a standard antigen solution.

The following examples further illustrate the present invention without limiting the technical scope of the present invention thereto. Those skilled in the art can readily add modifications and/or changes to the present invention on the basis of the description herein, and those modifications and/ or changes are also considered to fall within the technical scope of the present invention.

### EXAMPLES

### Example 1: Preparation of monoclonal antibodies against MTF

Membrane fragments were prepared from human cartilage cultured cells, and then membrane-bound components containing MTF were solubilized with phospholipase C specifically acting on GPI anchors.

Then, the solubilized membrane-bound components were applied on an immunoaffinity chromatograph using a Cellulofine gel to which human MTF-specific monoclonal antibody L235 (ATCC No. 8446-HB) had been immobilized so that human MTF was specifically bound to the chromatograph. MTF was eluted with 0.1 M glycine hydrochloride buffer (pH 2.4) used as an eluent.

Balb/c mice were intraperitoneally injected with 10 µg of purified MTF as an immunizing antigen with Freund's complete adjuvant. Then, subcutaneous booster injections were repeated. The increase of the antibody titer of mouse antiserum against MTF was observed by an indirect competitive ELISA method using an MTF-immobilized microtiter plate. Then, antibody-producing cells and mouse myeloma cells (xAg8/6.5.3) were fused to give two hybridoma cell strains producing MTF-specific monoclonal antibodies (47-4E and 8-9C) based on the antibody titer against MTF measured by a similar indirect competitive ELISA method.

The resulting anti-MTF monoclonal antibodies 47-4E and 8-9C were tested for their specificities for different sources of MTF by comparing their specificities for MTF from cartilage cultured cells corresponding to the immunizing antigen source and MTF from melanoma cells. Human serum-derived transferrin was used as a negative control. The results are shown in Table 1.

**Table 1**

| Monoclonal antibody | Melanotransferrin from | | Transferrin from human serum |
|---|---|---|---|
| | cartilage cultured cells | melanoma cells | |
| 47-4E | 100% | 100% | 0% |
| 8-9C | 100% | 100% | 0% |

Table 1 shows that anti-MTF monoclonal antibodies of the present invention are specific for MTF irrespective of the source of MTF which is the target compound to be assayed.

### Example 2: Measurement of MTF using a sandwich enzyme immunoassay

The two anti-MTF monoclonal antibodies 47-4E and 8-9C obtained in Example 1 were used for measuring MTF by a sandwich enzyme immunoassay (double antibody method).
a) Preparing a plate having a primary antibody (anti-MTF monoclonal antibody 8-9C) immobilized thereon.
b) Blocking the immobilized plate.
c) Adding and reacting an MTF antigen.
d) Adding and reacting a secondary antibody (biotinylated anti-MTF monoclonal antibody 47-4E).
e) Adding alkaline phosphatase-labeled streptavidin.
f) Adding a p-nitrophenylphosphate solution.
g) Stopping the chromogenic reaction.

A washing step with PBS containing 0.1% Tween 20 was perfirmed between various steps.

Specifically, a microtiter plate was first coated by pipetting a 100 µl aliquot of 30 µg/ml of anti-MTF monoclonal antibody 8-9C obtained in Example 1 into each well. The plate was then incubated at 4°C overnight. Then, the immobilized plate was blocked with 20 mM phosphate buffer (pH 7.4) containing 1% (w/v) bovine serum albumin (BSA) and 0.15 M NaCl to prepare an assay plate.

Then, a 100 µl aliquot of 0-100 µg/ml MTF antigen prepared in Example 1 was added into each well and reacted at room temperature for 2 hours.

After reaction, the well was washed with PBS containing 0.1% Tween 20 and 2.5 µg/ml of biotinylated anti-MTF monoclonal antibody 47-4E was added as a secondary antibody. Reaction was performed at room temperature for 2 hours. Then, 0.5 U/ml alkaline phosphatase-labeled streptavidin was added and similarly reacted at room temperature for 2 hours. After completion of the reaction, the well was washed with PBS containing 0.1% Tween 20 and a p-nitrophenylphosphate solution (Biotecx Laboratories Inc., USA) was added as a chromogenic substrate solution. Chromogenic reaction was performed at room temperature for 30 minutes and then stopped by adding 1N NaOH.

The absorbency at 415 nm was measured with a plate reader to determine color intensity. A calibration curve prepared with a standard melanotransferrin solution is shown in Fig. 1. As shown in Fig. 1, MTF can be measured by assay methods of the present invention at a level of about 1 ng/ml to about 80 ng/ml in the case of a sandwich method using anti-MTF monoclonal antibodies 47-4E and 8-9C.

### Example 3: Measurement of MTF in synovial fluid using anti-MTF antibodies

MTF levels in synovial fluid of arthritis patients were measured according to the procedure of the sandwich enzyme immunoassay of Example 2.

Specifically, the subjects were arthritis patients including 26 cases of osteoarthritis (OA), 46 cases of rheumatoid arthritis (RA) and 15 cases of traumatic joint injury (ACL). As negative controls, 2 cases of medial meniscus injury were also tested. The results are shown in Fig. 2.

Fig. 2 shows that MTF levels in synovial fluid in any types of arthritis including osteoarthritis, rheumatoid arthritis and traumatic joint injury are significantly higher than in control medial meniscus injury. This indicates that MTF levels in synovial fluid are useful for early finding and diagnosis of arthritis.

It was also found that MTF level distributions are significantly different among osteoarthritis, rheumatoid arthritis and traumatic joint injury. Specifically, MTF level distributions were an average of about 3.15 ng/ml (SD ± 2.79) and a maximum of about 8 ng/ml in osteoarthritis (OA), an average of about 11.30 ng/ml (SD ± 11.82) and a maximum of about 52 ng/ml in rheumatoid arthritis (RA), and an average of about 9.88 ng/ml (SD ± 5.29) and a maximum of about 25 ng/ml in traumatic joint injury (ACL). In contrast, the distribution in negative control medial meniscus injury was an average of about 1.14 ng/ml (SD ± 1.21).

### Example 4: Correlation between serum MTF levels and synovial fluid MTF levels

In addition to synovial fluid, serum was also collected from 26 cases of osteoarthritis, 46 oases of rheumatoid arthritis and 15 cases of traumatic joint injury. MTF levels in serum were measured and compared with MTF levels in synovial fluid.

The results are shown in Fig. 3. As shown in Fig. 3, a positive correlation was found between MTF levels in serum and MTF levels in synovial fluid. More specifically, MTF levels in serum and MTF levels in synovial fluid are correlated by a correlation coefficient of about 0.694, with the relational expression: serum MTF = synovial fluid MTF x 0.664 + 1.71.

### EFFEECT OF THE INVENTION

The present invention revealed the relation between MTF and arthritis. MTF assay methods of the present invention enables convenient, rapid and economic finding and diagnosis of a wide range of arthritis by measuring MTF levels in biological samples.

The present invention also revealed significant differences in MTF levels among three types of arthritis. i.e. osteoarthritis, rheumatoid arthritis and traumatic joint injury, and first succeeded in discriminating among the three types.

## Claims

1. A method for assaying arthritis-related melanotransferrin comprising;
i) measuring the melanotransferrin level in a biological sample; and
ii) indicating arthritis if the melanotransferrin level in the biological sample is significantly higher than in a control non-arthritis biological sample.

2. The method of Claim 1 wherein the biological sample is synovial fluid or serum.

3. The method of Claim 1 or 2 comprising indicating arthritis if the melanotransferrin level in synovial fluid is about 2 ng/ml or more or if the melanotransferrin level in serum is about 5 ng/ml or more.

4. The method of any one of Claims 1 to 3 comprising indicating any one of rheumatoid arthritis, osteoarthritis and traumatic joint injury if the melanotransferrin level in synovial fluid is about 2 ng/ml or more, indicating rheumatoid arthritis or traumatic joint injury if it is about 8 ng/ml or more, and indicating rheumatoid arthritis if it is about 25 ng/ml or more.

5. The method of any one of Claims 1 to 4 comprising measuring the melanotransferrin level in a biological sample by a sandwich immunoassay using a melanotransferrin-specific antibody.

6. The method of Claim 5 comprising using anti-melanotransferrin monoclonal antibodies 47-4E and 8-9C.

7. A reagent for use in the method of any one of Claims 1 to 6 comprising a melanotransferrin-specific antibody.

8. The reagent of Claim 7 comprising anti-melanotransferrin monoclonal antibodies 47-4E and 8-9C.

9. The reagent of Claim 7 or 8 wherein the melanotransferrin-specific antibody is labeled.

10. A kit for assaying melanotransferrin comprising the reagent of any one of Claims 7 to 9 in a suitable container.

11. The kit of Claim 10 further comprising a plate for immobilizing an antibody, a chromogenic substrate reagent or a labeled antibody.
